# EUROPÄISCHE PATENTSCHRIFT

(11) **EP 0 891 747 B1**
(45) Veröffentlichungstag und Bekanntmachung des Hinweises auf die Patenterteilung: **03.09.2003**
(21) Anmeldenummer: 98110273.4
(22) Anmeldetag: 05.06.1998
(51) Int. Cl.: A61B 17/70

(54) **Wirbelsäulenfixateur**
Spinal fixator
Fixateur de rachis

(30) Priorität: 18.07.1997 DE 29712697 U
(43) Veröffentlichungstag der Anmeldung: 20.01.1999
(73) Patentinhaber: TORNIER Vertriebs- und Beratungsgesellschaft für Medizintechnik mbH, 51399 Burscheid (DE)
(72) Erfinder: Bongartz, Robert, 41464 Neuss (DE); Kluger, P., Dr. med., 89155 Erbach (DE)
(74) Vertreter: Selting, Günther, Dipl.-Ing.

(56) Entgegenhaltungen:
- EP-A- 0 641 548
- FR-A- 2 731 344
- FR-A- 2 743 290
- US-A- 4 987 892
- US-A- 5 282 862

## Beschreibung

Die Erfindung betrifft einen Wirbelsäulenfixateur, mit dem zwei oder mehr Wirbelkörper der Wirbelsäule verbunden werden können, um beispielsweise einen oder mehrere andere Wirbelkörper zu überbrücken oder Repositionen von Fehlstellungen der Wirbelkörper vorzunehmen.

Aus DE 195 34 136 A1 ist ein Wirbelsäulenfixateur bekannt, der eine in einen Wirbelknochen einschraubbare Verankerungsschraube aufweist. Der Kopf der Verankerungsschraube hat eine mit einer entsprechenden Gegenanlagefläche versehene Klemmvorrichtung, die rotatorisch einstellbar befestigt wird. Die Klemmvorrichtung hält ihrerseits einen stabförmigen Längsträger, der die Verbindung zu einer ebenfalls mit einer Klemmvorrichtung ausgestatteten weiteren Verankerungsschraube vornimmt. Um die Steifheit der Zusammenfügung über längere Zeit beizubehalten und eine perfekte Rotations- und Translationsblockierung des Längsträgers zu gewährleisten, sind die Klemmvorrichtung und der Längsträger mit nicht zusammenpassenden Gewinden unterschiedlicher Ganghöhen ausgestattet, wodurch sich beide Gewinde beim Spannen gegeneinander verklemmen. Es hat sich jedoch herausgestellt, daß ein solcher Eingriff nicht zusammenpassender Gewinde, verschiedene Nachteile hat und auch nicht die gewünschte Rotationssicherheit gewährleisten.

In US 4,987,892 A ist ein Wirbelsäulenfixateur nach dem Oberbegriff des Patentanspruchs 1 beschrieben, der eine Verankerungsschraube, einen stabförmigen Längsträger und eine den Längsträger umspannende Klemmvorrichtung aufweist. Die Klemmvorrichtung ist nach Art einer Rohrschelle ausgebildet, und sie hat zwei voneinander beabstandete, durch einen nachgiebigen Bogenabschnitt verbundene Schenkel, die durch eine Schraube gegeneinander gedrückt werden und dadurch den Längsträger festklemmen. Die Schenkel der Klemmvorrichtung sind zueinander symmetrisch. Der durch die Schenkel begrenzte Spalt mündet symmetrisch in das von dem Bogen abschnittumschlossene Loch ein.

Aus EP 0 641 548 A1 ist ein Wirbelsäulenfixateur bekannt, bei dem die Anlagefläche des Kopfes der Verankerungsschraube schräg zur Längsachse des Gewindeteils dieser Schraube verläuft. Gegen die Anlagefläche wird mit einer Spannschraube eine Platte gesetzt, die am Ende des Längsträgers befestigt ist. Hierbei muss der Längsträger auf Maß vorgefertigt werden. Der Längsträger durchquert die Achse der Spannschraube, wodurch die Position des Längsträgers vorgegeben ist.

Der Erfindung liegt die Aufgabe zugrunde, einen Wirbelsäulenfixateur zu schaffen, der eine Anordnung des Längsträgers nahe an einem Wirbelkörper ermöglicht und der eine hohe Rotationsstabilität des Längsträgers gewährleistet.

Die Lösung dieser Aufgabe erfolgt erfindungsgemäß mit den im Patentanspruch 1 angegebenen Merkmalen.

Bei dem erfindungsgemäßen Wirbelsäulenfixateur ist die den Längsträger an der Verankerungsschraube festhaltende Klemmvorrichtung nach Art einer modifizierten Rohrschelle ausgebildet. Sie weist zwei voneinander beabstandete, durch einen nachgiebigen Bogenabschnitt verbundene Schenkel auf. Hierdurch wird beim Festziehen einer Spannschraube die Rohrschelle unter Verformung des nachgiebigen Bogenabschnitts eng um den Längsträger herumgezogen und verspannt. Die Elastizität der Klemmvorrichtung bewirkt, daß die Schenkel auseinandergedrückt werden. Somit stehen die Schenkel und die sie zusammendrückende Spannschraube ständig unter einer elastischen Spannung, wodurch ein Lockern der Spannschraube verhindert wird.

Die Erfindung basiert auf dem Gedanken, daß die bisherigen Bemühungen der Erzielung einer rotationsfreien Verbindung durch Aufrauhung der gegeneinanderliegenden Teile nicht den erhofften Erfolg brachten und daß bessere Ergebnisse erzielt werden können, wenn zwei glatte Teile fest und mit einer gewissen Elastizität gegeneinandergedrückt gehalten werden. Wichtig ist dabei, daß nicht lediglich eine bloße Klemmung stattfindet, sondern ein umfangsmäßiges Umschnüren des Längsträgers. Hierzu muß der Bogenabschnitt der Klemmvorrichtung die nötige Nachgiebigkeit bzw. Elastizität aufweisen.

Gemäß einer bevorzugten Ausgestaltung der Erfindung ist die Klemmvorrichtung einstückig ausgebildet, wobei der Bogenabschnitt integral in die beiden Schenkel übergeht. Die Schenkel sind wegen ihrer größeren Materialstärke nahezu starr, während der Bogenabschnitt verformbar ist.

Eine besonders wirksame Klemmwirkung ergibt sich, wenn die Schenkel der Klemmvorrichtung einen Spalt begrenzen, der asymmetrisch in den Bogenabschnitt einmündet. Asymmetrisch bedeutet hierbei, daß die Mittelebene des Spaltes nicht durch den Mittelpunkt des kreisförmigen Bogenabschnitts hindurchgeht. Dabei entstehen an dem Übergang des Spaltes in die Enden des Bogenabschnitts Spitzen, die sich beim Zusammendrücken der Schenkel in das Material des Längsträgers eingraben. Eine weitere Wirkung dieser Konstruktion besteht darin, daß der eine Schenkel, der an dem Kopf der Verankerungsschraube anliegt, gewissermaßen eine Basisplatte bildet und daß der gegenüberliegende Schenkel, gegen den der Kopf der Spannschraube drückt, während des Spannens eine definierte Schwenkbewegung ausführt. Bei symmetrischer Anordnung der Schenkel würden sich die Spannkräfte und Verformungen undefiniert auf beide Schenkel aufteilen.

Gemäß einer bevorzugten Ausführungsform der Klemmvorrichtung weist derjenige Schenkel, an dem der Kopf der Spannschraube angreift, eine Angriffsfläche auf, die schräg zu der Innenfläche dieses Schenkels verläuft. Werden beide Schenkel gegeneinandergedrückt, dann stellt sich diese Angriffsfläche (im gespannten Zustand) etwa parallel zu dem anderen Schenkel ein. Dadurch wird eine gleichmäßige Flächenverteilung der von der Spannschraube ausgeübten Kraft erzielt.

Die Innenfläche des Bogenabschnitts der Klemmvorrichtung und/oder die Oberfläche des Längsträgers sind vorzugsweise glatt ausgebildet. Hierdurch wird bei fester Klemmung ein vollkommener Formschluß erreicht, wodurch der Längsträger sowohl gegen Verdrehen als auch gegen Verschiebung in der Klemmvorrichtung mit hoher Kraft festgehalten wird.

Im folgenden wird unter Bezugnahme auf die Zeichnungen ein Ausführungsbeispiel der Erfindung näher erläutert.

Es zeigen:
- Fig. 1: einen Horizontalschnitt durch einen Wirbel der Wirbelsäule eines aufrechtstehenden Menschen mit eingesetztem Wirbelsäulenfixateur,
- Fig. 2: eine Seitenansicht der Klemmvorrichtung,
- Fig. 3: eine Unteransicht der Klemmvorrichtung in Richtung des Pfeiles III von Fig. 2, und
- Fig. 4: eine Draufsicht der Klemmvorrichtung aus Richtung des Pfeiles IV von Fig. 3.

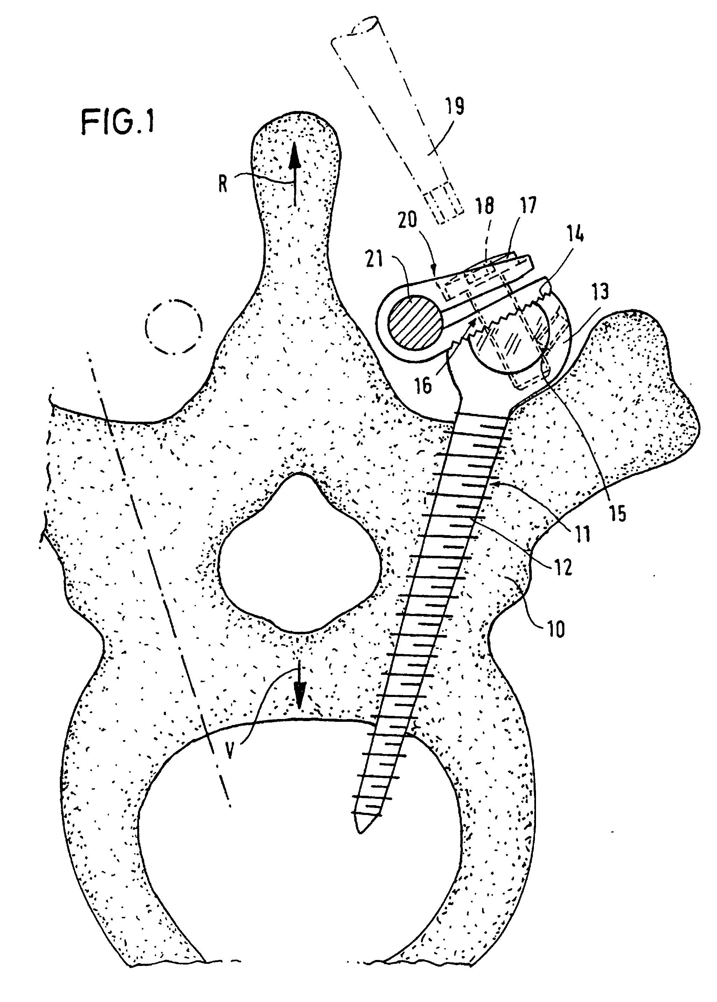

In Figur 1 ist ein horizontaler Querschnitt durch einen Wirbel 10 der Wirbelsäule dargestellt. Dabei weist der Pfeil V nach vorne, also in Richtung des Bauches und der Pfeil R nach hinten. Der Fixateur weist eine verankerungsschraube 11 auf, die als Knochenschraube ausgebildet ist und mit einem Gewindeteil 12 in den Wirbelknochen eingeschraubt ist. Der Gewindeteil 12 ist als selbstschneidendes Gewinde ausgebildet und erstreckt sich bis zu dem Kopf 13 der Verankerungsschraube 12. Dieser Kopf 13 ist eine Kugel, die entlang einer Anlagefläche abgeschnitten ist. Die Anlagefläche 14 ist nicht eben, sondern in ihr ist eine sternförmige Rillenkontur ausgebildet. Die Anlagefläche 14 verläuft schräg zu der Achse des Gewindeteils 12, und zwar unter einem Winkel von etwa 30°. Um die Verankerungsschraube 12 mit einem Werkzeug drehen zu können, sind an entgegengesetzten Seiten des Kopfes 13 parallelverlaufende Abflachungen vorgesehen, an denen das Werkzeug angreifen kann. Im Kopf 13 der Verankerungsschraube 12 befindet sich eine Gewindebohrung 15, deren Achse mittig und senkrecht durch die Anlagefläche 14 hindurchgeht. In diese Gewindebohrung 15 ist eine Spannschraube 16 eingeschraubt. Der Kopf 17 der Spannschraube 16 ist mit einem Innensechskant 18 versehen, um mit einem Werkzeug 19 die Spannschraube 16 drehen zu können.

An dem Kopf 13 der Verankerungsschraube 12 ist mit einer Klemmvorrichtung 20 ein stabförmiger Längsträger 21 befestigt. Dieser Längsträger erstreckt sich rechtwinklig zu dem Gewindeteil 12. Eine andere Stelle des Längsträgers 21 (unterhalb oder oberhalb der Zeichnungsebene von Fig. 1) ist über eine weitere Klemmvorrichtung 20 mit einer anderen Verankerungsschraube verbunden, die in einen anderen Wirbelknochen eingesetzt ist. Somit verbindet der Längsträger zwei (oder mehr) Wirbelknochen 10 untereinander, um diese relativ zueinander zu fixieren. Der Längsträger 21 ist eine zylindrische Stange mit glatter Oberfläche. Der Längsträger besteht ebenso wie die Verankerungsschraube 12 und die Klemmvorrichtung 20 aus Implantatstahl bzw. Titan.

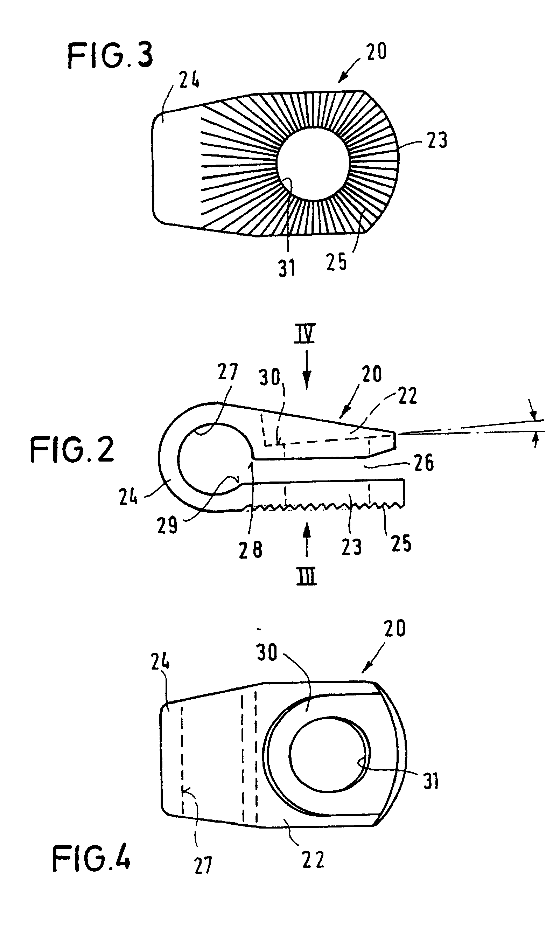

Die Klemmvorrichtung 20 ist in den Fign. 2 bis 4 im Detail dargestellt. Sie besteht aus einer einstückigen Klammer mit zwei Schenkeln 22,23, welche durch einen Bogenabschnitt 24 von verringerter Materialstärke verbunden sind. Die einander zugewandten Innenseiten der beiden Schenkel 22 und 23 verlaufen im ungespannten Zustand gemäß Fig. 2 parallel zueinander. Der eine Schenkel 23, der den Basisschenkel bildet, weist an seiner Außenfläche eine geriffelte Angriffsfläche 25 auf, die an der ebenfalls geriffelten Anlagefläche 14 des Kopfes 13 der Verankerungsschraube 11 anliegt, wobei die Riffelungen derart ausgebildet sind, daß sie verzahnend ineinandergreifen, so daß nach dem Festziehen der Spannschraube 16 die Klemmvorrichtung 20 nicht relativ zu dem Kopf 13 gedreht werden kann.

Die Schenkel 22,23 schließen einen Spalt 26 ein, der gemäß Figur 2 asymmetrisch zu dem von dem Bogenabschnitt 24 umschlossenen Loch 27 verläuft und in dieses einmündet. Das Loch 27 hat eine zylindrische glatte Innenfläche. Die Breite des Spaltes 26 beträgt etwa ein Drittel des Durchmessers des Lochs 27. Der Spalt 26 mündet ausschließlich in die untere Hälfte des Lochs 27 ein, so daß die Spaltmündung ausschließlich im Bereich der unteren Hälfte des Lochquerschnitts liegt. An der Mündung des Spaltes 12 in das Loch 27 befinden sich scharfe Kanten 28,29, die sich beim Spannen in die Oberfläche des Längsträgers eindrücken.

Der obere Schenkel 22 der Klemmvorrichtung 20 weist eine Vertiefung auf, deren Bodenfläche eine Angriffsfläche 30 bildet, welche unter einem kleinen Winkel von etwa 5° nach außen hin ansteigt. Gegen die Angriffsfläche 30 drückt der Kopf 17 der Spannschraube 16, wodurch der Schenkel 22 zum Schenkel 23 hin verschwenkt wird, so daß im gespannten Zustand die Angriffsfläche 30 etwa parallel zum Schenkel 23 verläuft. Durch die Mitte der Angriffsfläche 30 und durch den Schenkel 23 geht ein Loch 31 hindurch, durch das der Schaft der Spannschraube 16 gesteckt wird.

Der kreisförmige Bogenabschnitt 24 wird durch eine parallel zu dem Schenkel 23 verlaufende Mittelebene in zwei gleiche Hälften unterteilt. Der Spalt 26 befindet sich ausschließlich in der unteren Hälfte. Der Bogenabschnitt 24 bildet einen Federungsabschnitt, in dem sich die Schelle bei einem Druck gegen den Schenkel 22 verformt. Die Schenkel 22 und 23 sind dagegen starr.

Der Wirbelsäulenfixateur bietet verschiedene Möglichkeiten der Einstellung und Justierung des Verlaufs des Längsträgers 21. So kann nach dem Lösen der Spannschraube 16 die Klemmvorrichtung 20 um die Achse der Spannschraube herum gedreht und einstellt werden. Hierbei ist eine Verstellung des Längsträgers sowohl in Längsrichtung als auch in Querrichtung möglich, um eine Anpassung an lordotische und kyphotische Krümmungen der Wirbelsäule zu erreichen. Ferner kann bei der Justierung auch die Verankerungsschraube 11 noch gedreht werden, wodurch ebenfalls eine lordotische Verschiebung und eine Verschwenkung des Längsträgers erfolgt. Durch Verschwenken der Klemmvorrichtung um die Achse der Spannschraube 16 ist eine Höhenanpassung möglich.

Durch den schrägen Verlauf der Anlagefläche 14 der Verankerungsschraube wird vermieden, daß die Spannschraube 16 koaxial zu der Verankerungsschraube 11 ausgerichtet ist und daß beim Drehen der Spannschraube die Verankerungsschraube unbeabsichtigt verstellt werden kann. Dadurch, daß der Längsträger 21 nicht auf die Spannschraube 16 zuläuft, ergeben sich die oben aufgezeichneten unterschiedlichen Einstellmöglichkeiten.

## Patentansprüche

1. Wirbelsäulenfixateur mit einer Verankerungsschraube (11), die einen Gewindeteil (12) und einen Kopf (13) aufweist, wobei der Kopf (13) mit einer Anlagefläche (14) versehen ist, einem stabförmigen Längsträger (21) und einer den Längsträger (21) umspannenden Klemmvorrichtung (20), die mit einer Spannschraube (16) an der Anlagefläche (14) des Kopfes (13) mit einstellbarem Drehwinkel befestigt ist, wobei die Klemmvorrichtung (20) nach Art einer Rohrschelle ausgebildet ist und zwei voneinander beabstandete, durch einen nachgiebigen Bogenabschnitt (24) verbundene Schenkel (22,23) aufweist,
**dadurch gekennzeichnet,**
**dass** die Anlagefläche (14) schräg zu der Längsachse des Gewindeteils (12) verläuft und die Klemmvorrichtung (20) durch Drehen um die Achse der Spannschraube (16) in eine Stellung einstellbar ist, in der sich der Bogenabschnitt (24) und der Langsträger (21) auf derjenigen Seite der Anlagefläche (14) befinden, die den geringsten Abstand von dem Gewindeteil (12) hat.

2. Wirbelsäulenfixateur nach Anspruch 1, **dadurch gekennzeichnet, daß** die Klemmvorrichtung (20) einstückig ausgebildet ist.

3. Wirbelsäulenfixateur nach Anspruch 1 oder 2, **dadurch gekennzeichnet, daß** die Schenkel (22,23) der Klemmvorrichtung (20) einen Spalt (26) begrenzen, der asymmetrisch in das von dem Bogenabschnitt (24) umschlossene Loch (27) einmündet.

4. Wirbelsäulenfixateur nach Anspruch 3, **dadurch gekennzeichnet, daß** der Spalt (26) nur in die eine Hälfte des Lochs (27) mündet und durch eine parallel zu den Schenkeln (22,23) verlaufende Mittelebene in zwei Hälften unterteilt ist.

5. Wirbelsäulenfixateur nach einem der Ansprüche 1 bis 4, **dadurch gekennzeichnet, daß** derjenige Schenkel (22) der Klemmvorrichtung (20), an dem der Kopf (17) der Spannschraube (16) angreift, eine Angriffsfläche (30) aufweist, die schräg zu dem anderen Schenkel (23) verläuft.

6. Wirbelsäulenfixateur nach einem der Ansprüche 1 bis 5, **dadurch gekennzeichnet, daß** die Innenfläche des Bogenabschnitts (24) glatt ist.

7. Wirbelsäulenfixateur nach einem der Ansprüche 1 bis 6, **dadurch gekennzeichnet, daß** die von der Klemmvorrichtung (20) umfaßte Oberfläche des Längsträgers (21) glatt ist.

## Claims

1. Spinal fixator comprising an anchor bolt (11) having a threaded portion (12) and a head (13), wherein the head (13) is provided with a bearing surface (14), a rod-shaped longitudinal member (21) and a clamping device (20) embracing the longitudinal member (21), said clamping device (20) being fastened at an adjustable angle of rotation to the bearing surface (14) of the head (13) by means of a clamp bolt (16), wherein the clamping device (20) is configured in the kind of a pipe clamp and comprises two legs (22,23) arranged in a spaced relationship to each other and connected by a flexible arcuate section (24),
**characterized in that**
the bearing surface (14) extends at an inclination to the longitudinal axis of the threaded portion (12), and the clamping device (20) is adjustable by rotation about the axis of the clamp bolt (16) into a position in which the arcuate section (24) and the longitudinal member (21) are located on that side of the bearing surface (14) which has the smallest distance to the threaded portion (12).

2. Spinal fixator according to claim 1, **characterized in that** the clamping device (20) is configured as a single-piece device.

3. Spinal fixator according to claim 1 or 2, **characterized in that** the legs (22,23) of the clamping device (20) define a gap (26) asymmetrically entering the hole (27) enclosed by the arcuate section (24).

4. Spinal fixator according to claim 3, **characterized in that** the gap (26) enters only one half of the hole (27) and is divided into two halves by a center plane extending in parallel to the legs (22,23).

5. Spinal fixator according to one of claims 1 to 4, **characterized in that** that leg (22) of the clamping device (20) which is engaged by the head (17) of the clamp bold (16) comprises an engagement surface (30) extending at an inclination to the other leg (23).

6. Spinal fixator according to one of claims 1 to 5, **characterized in that** the inner surface of the arcuate section (24) is smooth.

7. Spinal fixator according to one of claims 1 to 6, **characterized in that** the surface of the longitudinal member (21) embraced by the clamping device (20) is smooth.

## Revendications

1. Fixateur de rachis comprenant une vis d'ancrage (11) qui présente une partie filetée (12) et une tête (13), la tête (13) étant dotée d'une surface d'appui (14), un longeron en forme de barre (21) et un dispositif de serrage (20) enserrant le longeron (21), qui est fixé avec une vis de serrage (16) sur la surface d'appui (14) de la tête (13) selon un angle d'orientation réglable, le dispositif de serrage (20) étant conçu sous la forme d'un collier de serrage et présentant deux branches (22, 23) espacées mutuellement, reliées par une section courbe élastique (24),
**caractérisé en ce que**
la surface d'appui (14) s'étend de manière inclinée par rapport à l'axe longitudinal de la partie filetée (12) et le dispositif de serrage (20) peut être réglé, en tournant autour de l'axe de la vis de serrage (16), dans une position dans laquelle la section courbe (24) et le longeron (21) se trouvent du côté de la surface d'appui (14) qui possède la plus faible distance par rapport à la partie filetée (12).

2. Fixateur de rachis selon la revendication 1, **caractérisé en ce que** le dispositif de serrage (20) est agencé d'un seul tenant.

3. Fixateur de rachis selon la revendication 1 ou 2, **caractérisé en ce que** les branches (22, 23) du dispositif de serrage (20) délimitent une fente (26) qui débouche de manière asymétrique dans le trou (27) cerné par la section courbe (24).

4. Fixateur de rachis selon la revendication 3, **caractérisé en ce que** la fente (26) ne débouche que dans une moitié du trou (27) et est divisée en deux moitiés par un plan médian courant parallèlement aux branches (22, 23).

5. Fixateur de rachis selon l'une quelconque des revendications 1 à 4, **caractérisé en ce que** la branche (22) du dispositif de serrage (20) sur laquelle s'applique la tête (17) de la vis de serrage (16) présente une surface d'attaque (30) qui s'étend de manière inclinée par rapport à l'autre branche (23).

6. Fixateur de rachis selon l'une quelconque des revendications 1 à 5, **caractérisé en ce que** la face interne de la section courbe (24) est lisse.

7. Fixateur de rachis selon l'une quelconque des revendications 1 à 6, **caractérisé en ce que** la surface extérieure du longeron (21) entourée par le dispositif de serrage (20) est lisse.
